# EUROPÄISCHE PATENTSCHRIFT

(11) **EP 2 700 665 B1**
(45) Veröffentlichungstag und Bekanntmachung des Hinweises auf die Patenterteilung: **05.12.2018**
(21) Anmeldenummer: 13180806.5
(22) Anmeldetag: 19.08.2013
(51) Int. Cl.: C08G 18/02, C08G 18/09, C08G 18/18, C08G 18/75, C07D 251/34

(54) **Verfahren zur Trimerisierung cycloaliphatischer Diisocyanate**
Method for trimerization of cycloaliphatic diisocyanates
Procédé de trimérisation de di-isocyanates cycloaliphatiques

(30) Priorität: 23.08.2012 EP 12181517
(43) Veröffentlichungstag der Anmeldung: 26.02.2014
(73) Patentinhaber: Covestro Deutschland AG, 51373 Leverkusen (DE)
(72) Erfinder: Halpaap, Reinhard, 51519 Odenthal (DE); Schneider, Mario, 51519 Odenthal (DE); Wilmes, Oswald, 51061 Köln (DE); Werner, Uwe, 50767 Köln (DE)
(74) Vertreter: Levpat

(56) Entgegenhaltungen:
- EP-A1- 0 524 501
- EP-A2- 1 170 283
- WO-A1-2005/087828
- DE-A1-102004 060 131
- LAAS H J ET AL: "ZUR SYNTHESE ALIPHATISCHER POLYISOCYANATE-LACKPOLYISOCYANATE MIT BIURET-, ISOCYANURAT- ODER URETDIONSTRUKTUR. ÖTHE SYNTHESIS OF ALIPHATIC POLYISOCYANATES CONTAINING BIURET, ISOCYANURATE OR URETDIONE BACKBONES FOR USE IN COATINGS", JOURNAL FUER PRAKTISCHE CHEMIE, WILEY VCH, WEINHEIM, DE, Bd. 336, Nr. 3, 1. Januar 1994 (1994-01-01), Seiten 185-200, XP000441642, ISSN: 1436-9966, DOI: 10.1002/PRAC.19943360302

## Beschreibung

Die Erfindung betrifft ein Verfahren zur Herstellung von Isocyanuratgruppen-haltigen Polyisocyanaten durch Trimerisierung von cycloaliphatischen Diisocyanaten unter Verwendung einer wenigstens ein quaternäres Ammoniumhydroxid enthaltenden Katalysatorlösung.

Monomerenarme Polyisocyanate werden inzwischen seit einigen Jahrzehnten als wertvolle Härter für Polyurethan-Beschichtungen und -Klebstoffe verwendet.

Hergestellt werden diese Härter in der Regel aus Diisocyanaten. Die Modifizierung dieser Diisocyanate zu Polyisocyanaten ist aus überwiegend zwei Gründen besonders vorteilhaft. Einerseits werden durch die Modifizierung meist Verzweigungen eingeführt, so dass die Polyisocyanate meist NCO-Funktionalitäten >2, oft im Bereich von 2,4 bis 4,5 aufweisen und so besonders gut zur Ausbildung hochvernetzter sehr beständiger Beschichtungen befähigt sind. Aber auch bei einer nur linearen Modifizierung, wie beispielsweise der Umsetzung mit Diolen zu linearen Prepolymeren werden den erhaltenen Härtern besondere Eigenschaften "mitgegeben", so z. B. die Fähigkeit besonders elastische Beschichtungen zu erzielen. Andererseits wird nach der eigentlichen Modifizierung der Diisocyanate in vielen Fällen das überschüssige monomere Diisocyanat abgetrennt und in den Modifizierungsprozess zurückgeführt. Die Polyisocyanate haben einen um Größenordnungen niedrigeren Dampfdruck als die durchaus flüchtigen Diisocyanate, so dass sie physiologisch deutlich weniger wirksam sind und arbeitshygienisch wesentlich einfacher zu verarbeiten sind.

In großen Mengen technisch eingesetzt werden als Diisocyanate Toluylendiisocyanat (TDl), Diphenylmethandiisocyanat (MDl), Hexamethylendiisocyanat (HDl), Isophorondiisocyanat (IPDI) und Dicyclohexylmethandiisocyanat (H₁₂-MDI). Neben diesen großtechnisch verfügbaren Diisocyanaten sind weitere Diisocyanate in technischen Mengen verfügbar. Solche sind neben den oben explizit genannten Diisocyanaten z. B. in Ullmann (Christian Six, Frank Richter, 2005 Wiley-VCH Verlag GmbH & Co. KGaA, Weinheim 10.1002/14356007.a14_611) beschrieben und haben häufig den Charakter von Spezialisocyanaten für ganz besondere Anwendungen.

Man unterscheidet zwischen aromatischen Diisocyanaten, bei denen die NCO-Gruppen direkt an einen aromatischen Ring gebunden sind wie bei TDI und MDI, und aliphatischen Diisocyanaten, die Isocyanatoalkyl-Gruppen aufweisen wie HDI, IPDI und H₁₂-MDI. Für hochwertige lichtechte Beschichtungen sind besonders die letztgenannten von Interesse, da sie nichtvergilbende farbstabile Beschichtungen liefern.

Die Oligomerisierung der Diisocyanate zu Polyisocyanaten ist bekannt und vielfach beschrieben, siehe z. B. H.J. Laas et al in J. Prakt. Chem. 336 (1994), 185ff. Die beschriebenen Oligomerisierungsverfahren unterscheiden sich in der Regel durch die Auswahl der einzusetzenden Diisocyanate, die Auswahl der Katalysatoren sowie die Wahl spezieller Reaktionsbedingungen.

Üblicherweise wird das Diisocyanat im Reaktionsgefäß vorgelegt und auf oder bis wenig unterhalb der Reaktionstemperatur vorgewärmt. Die zur Trimerisierung geeignete Katalysatorlösung wird dann über einen bestimmten Zeitraum kontinuierlich zugegeben. Nach Reaktionsbeginn ("Anspringen" der Reaktion) wird die stark exotherme Reaktion in der Regel gekühlt und durch Regelung der Katalysator-Dosierung und Kühlung die Reaktion so durchgeführt, dass der angestrebte Ziel-NCO-Gehalt der Rohlösung erreicht wird. Durch Zugabe eines chemischen Abstoppers wird noch vorhandener Katalysator desaktiviert. Das oligomere Polyisocyanat wird in einem nachfolgenden Verfahrensschritt von überschüssigem monomerem Diisocyanat abgetrennt. In aller Regel erfolgt diese Abtrennung durch Destillation in geeigneten Apparaturen, bevorzugt durch mehrstufige Destillation unter Einbeziehung mindestens einer Dünnschichtdestillation.

Die EP-A 0003765 beschreibt die Trimerisierung von IPDI mit Hilfe von quaternären Hydroxyalkyl substituierten Ammoniumhydroxiden als Katalysatoren bei Reaktionstemperaturen von 30 bis 90°C.

Nachteilig bei dieser Reaktionsführung ist es, dass sich, gerade bei den hier eingesetzten cycloaliphatischen Diisocyanaten und als Katalysatoren verwendeten quaternären Ammoniumhydroxid Verbindungen, sowie den sauren chemischen Abstoppern während und nach der Reaktion Katalysator-Salze bilden, die bei Reaktionstemperaturen unterhalb 90 °C nicht vollständig löslich sind. Diese als Trübung in Erscheinung tretenden Salze stören durch Belegung von Reaktorwandungen und Kühlflächen der Wärmetauscher den Wärmeübergang und erfordern ein häufiges Reinigen der Apparaturen. Die als Katalysatoren verwendeten quaternären Ammoniumhydroxid Verbindungen sind selbst thermolabil und werden bei erhöhten Temperaturen abgebaut, siehe z. B. H.J. Laas et al in J. Prakt. Chem. 336 (1994), 185ff. Dabei erfolgt Alkylierung des Anions des Salzes und Spaltung in tertiäre Amine. Die Abbauprodukte sind zum Teil flüchtig, zum Teil bilden sie ihrerseits katalytisch nicht mehr aktive Salze.

Aus EP 0524501 A1 ist ein Verfahren zur Herstellung von Isocyanuraten unter Verwendung von quartären Ammoniumhydroxiden bei einer Temperatur zwischen 90 und 100°C bekannt, wobei das erhaltene Olequ-merisat einen NCO-Gehalt von 35 Gew.-% aufweist, was einem Trimerisierungsgrad von 30 % entspricht. Der Prozess kann kontinuierlich oder diskontinuierlich durchgeführt werden.

In EP 1 170 283 A2 ist ein Verfahren zur Herstellung von Polyisocyanuraten auf Basis cycloaliphatischer Polyisocyanate beschrieben. Mit dem hier beschriebenen Verfahren wird unter anderem ein Trimerisat erhalten, welches einen NCO-Gehalt von 27,6 Gew.-% aufweist, was einem Trimerisierungsgrad von 25 Gew.-% entspricht, wobei anschließend das überschüssige Diisocyanat destillativ entfernt wird. Dieses Verfahren kann kontinuierlich oder diskontinuierlich durchgeführt werden, beispielsweise in einer Rührkesselkaskade.

WO 2005/087828 A1 beschreibt ein Verfahren zur Herstellung von Isocyanurat-Gruppen aufweisenden Polyisocyanaten und deren Verwendung. Bei dem Verfahren wird eine partielle Trimerisierung von (zyklo)-aliphatischen Diisocyanaten im Gegenwart mindestens eines Trimerisierungskatalysators durchgeführt. Als Trimerisierungskatalysatoren werden tetrasubstituierte Ammonium(α)-hydroxicarboxylate vorgeschlagen. Die nach diesem Verfahren hergestellten Isocyanurat-Gruppen aufweisenden Polyisocyanate können beispielsweise als Polyisocyanat-Komponente im Polyurethanlacken verwendet werden.

Aufgabe der vorliegenden Erfindung war es daher, ein diskontinuierliches (Batch) bzw. kontinuierliches Verfahren zur Verfügung zu stellen, bei dem die durch Ammoniumhydroxid Verbindungen katalysierte Trimerisierung von cycloaliphatischen Diisocyanaten so durchgeführt wird, dass den Betrieb der Anlage störende Feststoffablagerungen in den Apparaturen nicht auftreten. Insbesondere soll durch das spezielle Verfahren eine lange Betriebslaufzeit ohne Störungen und aufwändige Stillstände und notwendige häufige Reinigungsoperationen gewährleistet sein. Zudem sollte kein gegenüber der Trimerisierung bei Temperatur unterhalb 90°C deutlich erhöhter Katalysatorbedarf auftreten.

Diese Aufgabe wird erfindungsgemäß gelöst durch ein Verfahren zur Herstellung von Isocyanuratgruppen-haltigen Polyisocyanaten durch Trimerisierung von cycloaliphatischen Diisocyanaten gemäß Anspruch 1.

Es wurde überraschend gefunden, dass trotz der stark exothermen TrimerisierungsReaktion weitgehend auf externe Kühlung der Reaktionsmischung verzichtet und die Umsetzung bei Temperaturen > 90°C und • 140°C gut steuerbar durchgeführt werden kann. Überraschend ist insbesondere, dass trotz der relativ hohen Reaktionstemperatur der thermisch labile quaternäre Ammonium-Katalysator die Umsetzung ausreichend gut bis zum Erreichen des angestrebten NCO-Gehalts katalysiert und bei Reaktionsabbruch rasch thermisch so desaktiviert ist, dass man eine stabile Rohlösung ohne schleichenden weiteren NCO-Abfall erhält. Es wird kein gegenüber der Trimerisierung bei niedrigerer Temperatur unterhalb 90°C (z. B. bei 60-70 °C) deutlich erhöhter Katalysatorbedarf beobachtet. Oberhalb von 140°C tritt zu starke Deaktivierung des Katalysators auf, so dass eine deutlich erhöhte Menge an Katalysator nötig wird um den gewünschten Trimerisierungsgrad zu erreichen oder die Umsetzung aufgrund der raschen Katalysatorzersetzung gar nicht mehr startet.

Es hat sich gezeigt, dass die Reaktionstemperatur optimaler Weise > 90°C und • 140°C, bevorzugt > 90°C und • 135 °C und besonders bevorzugt • 95 °C und • 120 °C gewählt werden sollte.

Bei dieser Temperatur ist der Katalysator ausreichend stabil um den Trimerisierungsfortschritt aufrecht zu erhalten, ohne dass der oben beschriebene thermische Abbau des Katalysators schon einen wesentlichen Einfluss hat. Die gewählte Reaktionstemperatur ist andererseits so hoch, dass klare oder nur minimal trübe Polyisocyanat-Lösungen erhalten werden, bei denen Salz-Ablagerungen aus Katalysator-Abbauprodukten an Wärmetauschern, Reaktorwandungen oder Rohrleitungen nicht oder nur in sehr geringem Maße auftreten.

Diese Beobachtung ist überraschend, da die geringe Temperaturdifferenz zu einer üblichen Reaktionstemperatur von zum Beispiel 70 °C, wie sie typischerweise für eine ausreichend hohe Umsetzungsgeschwindigkeit gewählt wird, keinen so drastischen Effekt erwarten lässt.

Die Salzablagerungen werden überraschenderweise auch dann vermieden oder auf ein Minimum zurückgedrängt, wenn Diisocyanate mit durchaus erhöhten Gehalten an hydrolysierbaren Chlor-Verbindungen (HC-Gehalten) von z. Bsp. >150 - 500 ppm zum Einsatz kommen, wie sie in durch Phosgenierung der Diamine erhaltenen Diisocyanate vorkommen können.

Das Verfahren kann als Batch-Verfahren oder kontinuierlich in einem oder mehreren Rührkesseln durchgeführt werden, bevorzugt wird es kontinuierlich in einem oder mehreren Rührkesseln durchgeführt.

Durch das erfindungsgemäße Verfahren wird bevorzugt ein NCO-Gehalt erreicht, der einem Trimerisierungsgrad von 8 bis 30%, bevorzugt von 12 bis 24% entspricht.

Unter dem genannten Trimerisierungsgrad (T_{g}) versteht man den Anteil an im ursprünglichen Diisocyanat vorhandenen NCO-Gruppen (NCO₀), der bei der Oligomerisierung umgesetzt wurde. T_{g} = (NCO₀ - NCO) / NCO₀, wobei der Wert NCO der in der aktuellen Rohtrimerisat-Lösung vorhandene NCO-Gehalt ist.

Ein weiterer Gegenstand der Erfindung ist ein kontinuierliches Verfahren zur Herstellung von Isocyanuratgruppen-haltigen Polyisocyanaten durch Trimerisierung von cycloaliphatischen Diisocyanaten unter Verwendung einer wenigstens ein quaternäres Ammoniumhydroxid enthaltenden Katalysatorlösung in einer Rührkesselkaskade mit n Rührkesseln, dadurch gekennzeichnet, dass in mindestens n/2 oder (n/2 - 0,5) Kesseln so dosiert wird, dass die Reaktionstemperatur in mindestens n/2 oder (n/2 + 0,5) Kesseln bei > 90 °C und • 140°C, bevorzugt bei > 90 °C und • 135 °C und besonders bevorzugt • 95 °C und • 120 °C liegt, wobei für eine gerade Anzahl an Kesseln n/2 und für eine ungerade Anzahl an Kesseln (n/2 - 0,5) bzw. (n/2 + 0,5) gilt und n eine ganze Zahl von 2 bis 6 bedeutet.

Durch das vorstehende erfindungsgemäße Verfahren wird bevorzugt im n-ten Kessel ein NCO-Gehalt erreicht, der einem Trimerisierungsgrad von 8 bis 30%, bevorzugt von 12 bis 24% entspricht.

Für das erfindungsgemäße Verfahren geeignete Katalysatoren sind insbesondere die als sehr aktive Katalysatoren bekannten quaternären Ammoniumhydroxid Verbindungen (siehe H.J. Laas et al in J. Prakt. Chem. 336 (1994), 185ff). Besonders als Katalysatoren geeignet sind quaternäre Tetraalkylammonium-, gemischte Tetraalkyl/aralkylammonium- und Tetraaralkylammoniumhydroxide. Ganz besonders gut geeignet sind quaternäre Trialkylaralkylammoniumhydroxide, wie beispielsweise Benzyltrialkylammoniumhydroxide. Gut geeignet sind ferner Hydroxyalkyl-substituierte quaternäre Ammoniumhydroxide vom Cholin-Typ.

Bevorzugt wird als Katalysator wenigstens ein quaternäres Tetraalkylammoniumhydroxid und/oder ein quaternäres Trialkylaralkylammoniumhydroxid, bevorzugt wenigstens ein quaternäres Trialkylaralkylammoniumhydroxid eingesetzt. In einer speziellen Ausführungsform der Erfindung wird ausschließlich ein Tetraalkylammoniumhydroxid oder ein quaternäres Trialkylaralkylammoniumhydroxid als Katalysator, bevorzugt ausschließlich ein quaternäres Trialkylaralkylammoniumhydroxid eingesetzt.

Insbesondere bevorzugt wird als Katalysator wenigstens ein quaternäres Benzyltrialkylammoniumhydroxid eingesetzt.

Besonders bevorzugt wird ausschließlich ein quaternäres Benzyltrialkylammoniuhydroxid, ganz besonders bevorzugt ausschließlich Benzyltrimethylammoniumhydroxid eingesetzt.

In einer weiteren bevorzugten Ausführungsform der Erfindung wird als Katalysator wenigstens ein quaternäres Tetraalkylammonium- und/oder quaternäres gemischtes Tetraalkyl/aralkylammoniumhydroxid eingesetzt, dessen Alkylreste keine Hydroxylgruppen enthalten.

Die Katalysatoren werden erfindungsgemäß in Mengen von 0.005 - 1 Gew.-% und bevorzugt von 0.005 - 0.2 Gew-% bezogen auf die Menge des eingesetzten Diisocyanats verwendet. Die Mengenangaben beziehen sich auf den aktiven Katalysator selbst, d. h. das insbesonders eingesetzte quaternäre Ammoniumhydroxid. Die Katalysatoren können sowohl in reiner Form als auch wie oben beschrieben bevorzugt in gelöster Form eingesetzt werden, wobei Alkohole als Lösungsmittel bekanntermaßen cokatalytisch wirken.

Die als Katalysatorlösungsmittel bevorzugten Alkohole sind beliebige aliphatische Alkoho-le, wobei niedermolekulare Mono- oder Diole bevorzugt sind. Beispielhaft seien als Katalysatorlösungsmittel genannt: Methanol, Ethanol, Isopropanol, n-Butanol, 2-Ethylhexanol, 1,2-Dihydroxyethan, 1,2-Dihydroxypropan, 1,3- und 1,4-Dihydroxybutan, 1,6- und 2,5-Dihydroxyhexan, 2,2,4-Trimethyl-1,3-dihydroxypentan oder 2-Ethyl-1,3-dihydroxyhexan oder beliebige Gemische dieser Alkohole.

Die Katalysatoren werden, sofern sie nicht wie es weniger bevorzugt ist lösemittelfrei eingesetzt werden, in Konzentrationen von 0.1 - 10 Gew.-%, bevorzugt 0.2 - 8 Gew.-%, ganz besonders bevorzugt 1 - 5 Gew.-% verwendet, jeweils bezogen auf die Katalysatorlösung.

Für das erfindungsgemäße Verfahren werden als cycloaliphatische Diisocyanate erfindungsgemäß solche des Molekulargewichtsbereiches 166 bis 318 g/mol eingesetzt, nämlich 1,3- bzw. 1,4-Diisocyanatocyclohexan, 1,4-Diisocyanato-3,3,5-trimethylcyclohexan, 1,3-Diisocyanato-2(4)-methylcyclohexan, 1-Isocyanato-3,3,5-trimethyl-5-isocyanatomethylcyclohexan (Isophorondiisocyanat, IPDI), 1-Isocyanato-1-methyl-4(3)isocyanatomethylcyclohexan, 1,8-Diisocyanato-p-menthan, 4,4'-Diisocyanato-1,1'-bi(cyclohexyl), 4,4'-Diisocyanato-3,3'-dimethyl-1 ,1'-bi(cyclohexyl), 4,4'-Diisocyanato-2,2',5,5'-tetramethyl-1 ,1'-bi(cyclohexyl), 4,4'- und/oder 2,4'-Diisocyanatodicyclohexylmethan, 4,4'-Diisocyanato-3,3'-dimethyldicyclohexylmethan, 4,4'-Diisocyanato-3,3',5,5'-tetramethyldicyclohexylmethan, 1,3-Diisocyanatoadamantan, und 1,3-Dimethyl-5,7-diisocyanatoadamantan, sowie beliebige Gemische solcher Diisocyanate. Dabei ist es unerheblich auf welchem Weg die Diisocyanate hergestellt wurden. Dies kann durch Phosgenierung der Diamine in der Flüssigphase oder in der Gasphase, ebenso aber auch nach bekannten phosgenfreien Verfahren wie z. Bsp. der thermischen Urethanspaltung mit oder ohne Katalyse erfolgen.

Bevorzugte cycloaliphatische Diisocyanate für das erfindungsgemäße Verfahren sind IPDI und/oder 4,4'- und/oder 2,4'-Diisocyanatodicyclohexylmethan. Ganz besonders bevorzugt wird IPDI eingesetzt.

Die Reaktionslösung kann chemisch oder thermisch abgestoppt werden, wird jedoch bevorzugt thermisch abgestoppt.

Im Falle einer chemischen Abstoppung wird die Reaktionslösung durch Zugabe einer sauren Verbindung, einer Säure und/oder eines Alkylierungsmittels abgestoppt.

Die bevorzugte thermische Abstoppung erfolgt entweder bei der gewählten Reaktionstemperatur durch einfaches Nachrühren nach Abklingen der Reaktion oder aber durch Temperaturerhöhung um bis zu 50 °C und Nachrühren bei dieser Temperatur.

Bevorzugt wird die Abstopptemperatur um bis zu 20 °C gegenüber der Reaktionstemperatur leicht erhöht. Besonders bevorzugt wird die thermische Abstoppung bei Reaktionstemperatur durchgeführt.

Nach Beendigung der Trimerisierungsreaktion liegt als Reaktionsprodukt eine Lösung des Isocyanuratgruppen-aufweisenden Polyisocyanats in überschüssigem monomerem cycloaliphatischen Diisocyanat vor.

In einer bevorzugten Ausführungsform der Erfindung wird in einem nachgeschalteten Schritt von diesem Reaktionsgemisch noch vorhandenes monomeres cycloaliphatisches Diisocyanat destillativ abgetrennt. Dies erfolgt vorzugsweise durch Dünnschichtdestillation im Vakuum, beispielsweise bei einem Druck von unter 1,0 mbar, vorzugsweise unter 0,5 mbar, besonders bevorzugt unter 0,2 mbar, unter möglichst schonenden Bedingungen, beispielsweise bei einer Temperatur von 100 bis 200 °C, vorzugsweise von 120 bis 180 °C. Die Abtrennung des monomeren Diisocyanats kann einstufig, bevorzugt aber mehrstufig erfolgen. So wird beispielsweise ein Fallfilmverdampfer als Vorverdampfer eingesetzt, bei dem die Hauptmenge des monomeren Diisocyanats abgetrennt wird, im nachgeschalteten Dünnschichtverdampfer erfolgt die Abtrennung weiteren Ausgangsdiisocyanats. Auf diese Weise erhält man hochwertige Polyisocyanate, die einen Gehalt an freiem Diisocyanat von maximal 0,5 Gew.-%, vorzugsweise von maximal 0,3 Gew.-%, aufweisen. Die anfallenden Destillate werden erneut zur Trimerisierung eingesetzt.

In einer weiteren Ausführungsform werden die monomeren cycloaliphatischen Diisocyanate durch Extraktion mit geeigneten, gegenüber Isocyanatgruppen inerten Lösungsmitteln, beispielsweise aliphatischen oder cycloaliphatischen Kohlenwasserstoffen wie Pentan, Hexan, Heptan, Cyclopentan oder Cyclohexan vom Reaktionsprodukt abgetrennt. Dieses Verfahren ist weniger bevorzugt.

Die so erhaltenen monomerenarmen Polyisocyanate werden als solche verwendet oder aber in geeigneten gegenüber NCO-Gruppen inerten Lösungsmitteln zu Polyisocyanat-Lösungen gelöst. Die nach dem erfindungsgemäßen Verfahren hergestellten Polyisocyanate werden in den bekannten Anwendungen wie z. B. 2-Komponenten Polyurethanlacken oder in Klebstoffanwendungen verwendet. Die so erhaltenen Polyisocyanate dienen darüber hinaus wie es bekannt ist als Ausgangsstoffe für weitere daraus hergestellte Derivate wie z. B. blockierte Polyisocyanate oder hydrophilierte Polyisocyanate.

Die Erfindung soll anhand der nachfolgenden Beispiele näher erläutert werden.

In den nachfolgenden Beispielen beziehen sich alle %-Angaben auf Gew.-% sofern nicht anders angegeben.

Die Bestimmung der NCO-Gehalte erfolgte titrimetrisch nach DIN EN ISO 11909.

Die Rest-Monomeren Gehalte an IPDI wurden nach DIN EN ISO 10283 gaschromatographisch mit internem Standard gemessen.

Die Viskositätsmessungen erfolgten mit einem Physica MCR 51 Rheometer der Fa. Anton Paar Germany GmbH (DE) nach DIN EN ISO 3219, sofern nicht anders angegeben.

Die Bestimmung der Hazen-Farbzahl erfolgt nach DIN EN 1557.

### Beispiele:

Herstellung der Katalysatorlösung: Eine 40%ige Lösung von Trimethylbenzylammoniumhydroxid (Triton B) in Methanol wurde mit n-Butanol auf einen Wirkstoffgehalt bezogen auf Triton B von 5 % verdünnt.

### Beispiel 1, kontinuierlich, erfindungsgemäß.

Isophorondiisocyanat wurde zunächst entgast. Dazu wurde das IPDI kontinuierlich in eine Entgasungskolonne eindosiert. Bei einer Dampfbeheizung mit 2,0 bar Dampf und einem Vakuum von 0,3 mbar wurden 10 % der Einsatzmenge als Destillat abgenommen und zurückgeführt, der Rest lief als Sumpfprodukt in eine Vorlage zum Einsatz für die Trimerisierung.

Zur Trimerisierung wurde das wie beschrieben entgaste Isophorondiisocyanat kontinuierlich in eine Kaskade aus 3 gerührten Reaktoren eindosiert. Reaktor 1 wurde auf 95 °C temperiert, Behälter 2 und 3 wurden auf 115°C eingeregelt. Die Dosiergeschwindigkeit wurde so geregelt, dass sich eine Verweilzeit pro Reaktor von jeweils 1 Stunde ergab. Durch kontinuierliche Dosierung von Katalysatorlösung, die ca. 80 ppm Triton B (Wirkstoff) bezogen auf die Einfahrmenge Isophorondiisocyanat entsprach, wurde im ersten Reaktor die Trimerisierung gestartet. Die Katalysatormenge wurde so eingeregelt, dass der ursprüngliche NCO-Gehalt von mindestens 37,0% auf 32,5% abnahm. Im zweiten Reaktor wurde ebenfalls Katalysatorlösung kontinuierlich zudosiert, die ca. 20 ppm Triton B (Wirkstoff) bezogen auf die Einfahrmenge Isophorondiisocyanat entsprach. Hier wurde die Menge so geregelt, dass der NCO-Gehalt weiter abnahm bis auf einen Gehalt von 30,5 % (Trimerisierungsgrad: 17%) im dritten Reaktor.

Während der Reaktionszeit wurde kontinuierlich trockener Stickstoff in Reaktor 1 über ein Tauchrohr eindosiert. Die Menge entsprach pro Stunde 20 % des Behältervolumens.

Vom so erhaltenen Rohtrimerisat wurde anschließend das überschüssige Isophorondiisocyanat destillativ abgetrennt. Das aus der Reaktion erhaltene Rohtrimerisat wurde in die zweistufige Destillation eingespeist. Die erste Stufe war ein Fallfilmverdampfer mit Umpumpkreislauf, die zweite Stufe ein Dünnschichtverdampfer. Die Destillation wurde bei 150-180 °C und einem Druck von 0,1 - 10 mbar betrieben. Nach Vorkonzentration in der ersten Destillationsstufe auf einen Gehalt an Isophorondiisocyanat von ca. 10% betrug der Restgehalt an Isophorondiisocyanat nach Destillation 0,5%. Das als Sumpfprodukt der Destillation erhaltene Harz wurde in einem Rührbehälter in Butylacetat gelöst. Die 70%ige Harzlösung lieferte folgende Kenndaten: 11,9 % NCO-Gehalt, Viskosität 600 mPas (23°C), Monomergehalt 0,35%, Hazen-Farbzahl 25.

Das im Destillationsschritt wiedergewonnene Isophorondiisocyanat wurde der Reaktion wieder zugeführt.

Die Temperaturführung der exothermen Trimerisierung in der Rührkesselkaskade erfolgte durch Zulauftemperatur, Zulaufgeschwindigkeit, Katalysatordosierung sowie außenliegende Heizung und Kühlung. Die Umsetzung kann kontinuierlich über einen Zeitraum von mehr als 12 Monaten betrieben werden, ohne dass Reinigungsstillstände erforderlich sind. Nach Begutachtung bei Anlagenstillstand waren die Reaktorinnenwandungen ohne wesentlichen Belag.

### Vergleichsbeispiel 2, kontinuierlich, nicht erfindungsgemäß

Im Vergleichsbeispiel wurde wie oben im Beispiel 1 beschrieben verfahren, mit dem Unterschied, dass Reaktor 1 ist auf 60 °C temperiert war und Behälter 2 und 3 während der Trimerisierungsreaktion auf eine Temperatur zwischen 65 und 75 °C durch innenliegende Wärmetauscher und außenliegende Kühlung temperiert wurden. Die Dosiergeschwindigkeit des IPDI, sowie die kontinuierliche Dosierung der Katalysatorlösung in Reaktor 1 und 2 entsprach den im Beispiel 1 beschriebenen Bedingungen. Die Katalysatormenge wurde so eingeregelt, dass der ursprüngliche NCO-Gehalt von mindestens 37,0% bis auf einen Gehalt von 30,5 % (Trimerisierungsgrad: 17%) im dritten Reaktor abnahm. Da der Katalysator wegen der relativ moderaten Reaktionstemperatur auch im dritten Reaktionskessel noch aktiv war, wurde die IPDI-Trimer-Rohlösung durch Zugabe von ca. 50 Gew.-% Dibutylphosphat (DBP) bezogen auf eingesetzten Katalysator Triton B abgestoppt. Während der Reaktionszeit wurde wie in Beispiel 1 beschrieben kontinuierlich trockener Stickstoff in Reaktor 1 über ein Tauchrohr eindosiert.

Die Umsetzung wurde kontinuierlich über einen längeren Zeitraum durchgeführt. Nach 7 bis 14 Tagen Laufzeit wurde eine deutlich verminderte Kühlleistung beobachtet. Nach Begutachtung bei Anlagenstillstand waren die Reaktorinnenwandungen und Wärmetauscher massiv mit festen Ablagerungen aus polymerem Polyisocyanat, sowie Ammoniumchloriden als Katalysator-Abbauprodukten belegt. Die Anlage muss in regelmäßigen Abständen mindestens monatlich gereinigt werden.

### Beispiel 3, Batch, erfindungsgemäß

1200 g Isophorondiisocyanat (IPDI) wurden in einem Mehrhalskolben mit Rührer, Rückflusskühler, Stickstoffzuführung und Vakuum-Anschluss sowie einer Katalysatordosierung vorgelegt. Es wird dreimal für 15 min bei Raumtemperatur im Vakuum (0,3 mbar) entgast und jeweils mit Stickstoff (N₂) belüftet. Man heizt mit einem Ölbad unter leichter N₂-Überleitung auf 95 °C Innentemperatur auf. Bei dieser Temperatur beginnend wird die 5 %ige Katalysatorlösung (Dichte 0,82 g/ml) mit einer Dosierrate von 15 ml/h zugetropft. Nach ca. 10 min beginnt die exotherme Trimerisierungsreaktion. Die Temperatur steigt bis 110 °C und wird bei dieser Temperatur durch leichtes Kühlen, später Heizen gehalten. Nachdem 4 ml Katalysatorlösung dosiert sind (nach 16 min) wird die weitere Dosierung gestoppt. Nach 30 min ist ein NCO-Gehalt von 32,4 % erreicht (Trimerisierungsgrad 14%). Man dosiert weitere 0,5 ml Katalysatorlösung (Dosierrate 3 ml/h) und erreicht nach weiteren 10 min einen NCO-Gehalt von 31,7 % (T_{g} = 16%). Zur themischen Abstoppung wird 30 min bei 110-120 °C nachgerührt. Die Rohlösung ist klar, ohne Trübung und ohne Belag an der Kolbenwandung. Für die Umsetzung wurden 154 ppm Triton B (Wirkstoff) verbraucht.

Diese erhaltene klare Rohlösung mit einem NCO-Gehalt von 31,6 % (T_{g} = 16%) wird in einer zweistufigen kontinuierlichen Destillation mit Vorverdampfer und Dünnschichtverdampfer bei 180 °C/0,14 mbar und einer Dosiergeschwindigkeit der Rohlösung von 280 g/h von monomerem IPDI befreit. Man erhält 206 g Polyisocyanat-Harz, das mit Butylacetat bei 110 °C innerhalb 2,5 h Rühren zu einer 70%igen Lösung gelöst wird.

Man erhält ein 70%ig in Butylacetat gelöstes IPDI-Trimerisat mit folgenden Kenndaten: 11,8 % NCO-Gehalt, Viskosität 610 mPas (23°C), Monomergehalt 0,13%, Hazen-Farbzahl 17.

### Vergleichsbeispiel 4, Batch, niedrige Reaktionstemperatur, nicht erfindungsgemäß

Wie im erfindungsgemäßen Beispiel 3 beschrieben werden 1200 g IPDI durch Entgasen und mit N₂ Belüften vorbehandelt und auf 65 °C Innentemperatur geheizt. Bei dieser Temperatur werden 4,5 ml Katalysatorlösung bei einer Dosierrate von 12 ml/h zudosiert. Die exotherme Trimerisierungsreaktion wird durch mehrfaches Kühlen bei 70 - 75 °C Reaktionstemperatur gehalten. Bei Erreichen eines NCO-Gehalts von 30,7 % (T_{g} = 19%) werden zum Abstoppen 0,44 ml einer 25%igen Lösung von Dibutylphosphat in IPDI zugegeben (Dichte 1,05 g/ml). Die Innentemperatur fällt, man rührt 15 min bei 80 °C nach und erhält eine trübe Rohlösung mit einem NCO-Gehalt von 29,8 % (T_{g} = 21%). Für die Umsetzung wurden 154 ppm Triton B (Wirkstoff) verbraucht.

Die trübe Rohlösung wird wie in Bsp. 3 beschrieben in der zweistufigen Destillation mit Vorverdampfer / Dünnschichtverdampfer bei 180 °C/0,1 mbar und einer Dosiergeschwindigkeit der Rohlösung von 370 g/h destilliert. Das erhaltene klare Festharz wird 70%ig in Butylacetat gelöst, und man erhält eine klare Lösung mit folgenden Kenndaten: 11,7 % NCO-Gehalt, Viskosität 690 mPas (23°C), Monomergehalt 0,09%, Hazen-Farbzahl 30.

Nach der Destillation der Rohlösung findet sich an der Innenwandung des Reaktionskolbens ein trüber Belag.

### Vergleichsbeispiel 5, Batch, hohe Reaktionstemperatur, nicht erfindungsgemäß

Wie im erfindungsgemäßen Beispiel 3 beschrieben werden 300 g IPDI durch Entgasen und mit N₂ Belüften vorbehandelt und auf 140 °C Innentemperatur geheizt. Beginnend mit einer Dosierrate von 2,0 ml/h werden zunächst 0,5 ml (70 ppm Triton B) bei 142 °C zugegeben. Man erhöht die Dosierrate bei 142 - 145 °C auf 4,0 ml/h und gibt insgesamt 2,0 ml (280 ppm Triton B) zu. Der NCO-Gehalt fällt nur leicht auf einen Wert von 36,3 %. Zur Sicherheit wird 1 ml einer 25 %igen Dibutylphosphatlösung in IPDI zum Abstoppen zugegeben und man lässt abkühlen. Trotz der nahezu doppelten Menge an Katalysator bezogen auf das erfindungsgemäße Beispiel 3 und Vergleichsbeispiel 4 kommt keine Trimerisierungsreaktion in Gang.

## Patentansprüche

1. Verfahren zur Herstellung von Isocyanuratgruppen-haltigen Polyisocyanaten durch Trimerisierung von cycloaliphatischen Diisocyanaten unter Verwendung einer wenigstens ein quaternäres Ammoniumhydroxid enthaltenden Katalysatorlösung, **dadurch gekennzeichnet, dass** die Katalysatorlösung so zudosiert wird, dass die Reaktionstemperatur bei > 90 °C und ≤ 140°C liegt, wobei als cycloaliphatische Diisocyanate 1,3- bzw. 1,4-Diisocyanatocyclohexan, 1,4-Diisocyanato-3,3,5-trimethylcyclohexan, 1,3-Diisocyanato-2(4)-methylcyclohexan, 1-Isocyanato-3,3,5-trimethyl-5-isocyanatomethylcyclohexan (Isophorondiisocyanat, IPDI), 1-Isocyanato-1-methyl-4(3)isocyanatomethylcyclohexan, 1,8-Diisocyanato-p-menthan, 4,4'-Diisocyanato-1,1'-bi(cyclohexyl), 4,4'-Diisocyanato-3,3'-dimethyl-1,1 '-bi(cyclohexyl), 4,4'-Diisocyanato-2,2',5,5'-tetramethyl-1,1 '-bi(cyclohexyl), 4,4'-Diisocyanatodicyclohexylmethan, 2,4'-Diisocyanatodicyclohexylmethan, 4,4'-Diisocyanato-3,3'-dimethyldicyclohexylmethan, 4,4'-Diisocyanato-3,3',5,5'-tetramethyl-dicyclohexylmethan, 1,3-Diisocyanato-adamantan, 1,3-Dimethyl-5,7-diiso-cyanatoadamantan, sowie beliebige Gemische dieser Diisocyanate eingesetzt werden und wobei 0.005 - 1 Gew.-%, bezogen auf die Menge des eingesetzten Diisocyanats, an quaternärem Ammoniumhydroxid als Katalysator eingesetzt werden.

2. Verfahren gemäß Anspruch 1, **dadurch gekennzeichnet, dass** die Katalysatorlösung so zudosiert wird, dass die Reaktionstemperatur bei > 90 °C und ≤ 135 °C.

3. Verfahren gemäß Anspruch 1, **dadurch gekennzeichnet, dass** die Katalysatorlösung so zudosiert wird, dass die Reaktionstemperatur bei ≥ 95 °C und ≤ 120 °C liegt.

4. Verfahren gemäß einem der Ansprüche 1 bis 3, **dadurch gekennzeichnet, dass** dieses kontinuierlich in einem oder mehreren Rührkesseln durchgeführt wird.

5. Verfahren gemäß einem der Ansprüche 1 bis 4, **dadurch gekennzeichnet, dass** ein NCO-Gehalt erreicht wird, der einem Trimerisierungsgrad von 8 bis 30 % entspricht.

6. Kontinuierliches Verfahren zur Herstellung von Isocyanuratgruppen-haltigen Polyisocyanaten durch Trimerisierung von cycloaliphatischen Diisocyanaten unter Verwendung einer wenigstens ein quaternäres Ammoniumhydroxid enthaltenden Katalysatorlösung in einer Rührkesselkaskade mit n Rührkesseln, **dadurch gekennzeichnet, dass** in mindestens n/2 oder (n/2 - 0,5) Kesseln so dosiert wird, dass die Reaktionstemperatur in mindestens n/2 oder (n/2 + 0,5) Kesseln bei > 90 °C und ≤ 140°C liegt, wobei für eine gerade Anzahl an Kesseln n/2 und für eine ungerade Anzahl an Kesseln (n/2 - 0,5) bzw. (n/2 + 0,5) gilt und n eine ganze Zahl von 2 bis 6 bedeutet.

7. Verfahren gemäß Anspruch 6, **dadurch gekennzeichnet, dass** im n-ten Kessel ein NCO-Gehalt erreicht wird, der einem Trimerisierungsgrad von 8 bis 30% entspricht.

8. Verfahren gemäß einem der Ansprüche 1 bis 7, **dadurch gekennzeichnet, dass** die Katalysatoren als Lösungen der Konzentrationen 0.1 - 10 Gew.-% bezogen auf die Katalysatorlösung, eingesetzt werden.

9. Verfahren gemäß einem der Ansprüche 1 bis 8, **dadurch gekennzeichnet, dass** als Katalysator wenigstens ein quaternäres Tetraalkylammoniumhydroxid und/oder ein gemischtes quaternäres Tetraalkyl/aralkylammoniumhydroxid und/oder ein quaternäres Tetraaralkylammoniumhydroxid eingesetzt wird.

10. Verfahren gemäß Anspruch 9, **dadurch gekennzeichnet, dass** als Katalysator wenigstens ein quaternäres Trialkylaralkylammoniumhydroxid eingesetzt wird.

11. Verfahren gemäß Anspruch 9, **dadurch gekennzeichnet, dass** als Katalysator wenigstens ein quaternäres Tetraalkylammoniumhydroxid und/oder ein quaternäres gemischtes Tetraalkyl/aralkylammoniumhydroxid eingesetzt wird, dessen Alkylreste keine Hydroxylgruppen enthalten.

12. Verfahren gemäß Anspruch 9, **dadurch gekennzeichnet, dass** als Katalysator ausschließlich ein quaternäres Benzyltrialkylammoniumhydroxid eingesetzt wird.

13. Verfahren gemäß Anspruch 9, **dadurch gekennzeichnet, dass** als Katalysator ausschließlich Benzyltrimethylammoniumhydroxid eingesetzt wird.

14. Verfahren gemäß einem der Ansprüche 1 bis 13, **dadurch gekennzeichnet, dass** als cycloaliphatisches Diisocyanat IPDI eingesetzt wird.

15. Verfahren gemäß einem der Ansprüche 1 bis 14, **dadurch gekennzeichnet, dass** die Reaktionslösung chemisch oder thermisch, bevorzugt thermisch abgestoppt wird.

16. Verfahren gemäß Anspruch 15, **dadurch gekennzeichnet, dass** die Reaktionslösung chemisch durch Zugabe einer sauren Verbindung, einer Säure und/oder eines Alkylierungsmittels abgestoppt wird.

17. Verfahren gemäß einem der Ansprüche 1 bis 16, **dadurch gekennzeichnet, dass** in einem nachgeschalteten Schritt noch vorhandenes monomeres cycloaliphatisches Diisocyanat destillativ von dem Polyisocyanat abgetrennt wird.

## Claims

1. Process for preparing polyisocyanates containing isocyanurate groups by trimerising cycloaliphatic diisocyanates using a catalyst solution comprising at least one quaternary ammonium hydroxide, **characterized in that** the catalyst solution is metered in such that the reaction temperature is > 90°C and ≤ 140°C, wherein the cycloaliphatic diisocyanates used are 1,3- or 1,4-diisocyanatocyclohexane, 1,4-diisocyanato-3,3,5-trimethylcyclohexane, 1,3-diisocyanato-2(4)-methylcyclohexane, 1-isocyanato-3,3,5-trimethyl-5-isocyanatomethylcyclohexane (isophorone diisocyanate, IPDI), 1-isocyanato-1-methyl-4(3)-isocyanatomethylcyclohexane, 1,8-diisocyanato-*p-*menthane, 4,4'-diisocyanato-1,1'-bi(cyclohexyl), 4,4'-diisocyanato-3,3'-dimethyl-1,1'-bi(cyclohexyl), 4,4'-diisocyanato-2,2',5,5'-tetramethyl-1,1'-bi(cyclohexyl), 4,4'-diisocyanatodicyclohexylmethane, 2,4'-diisocyanatodicyclohexylmethane, 4,4'-diisocyanato-3,3'-dimethyldicyclohexylmethane, 4,4'-diisocyanato-3,3',5,5'-tetramethyldicyclohexylmethane, 1,3-diisocyanatoadamantane, 1,3-dimethyl-5,7-diisocyanatoadamantane, and also any mixtures of said diisocyanates and wherein 0.005 - 1% by weight of quaternary ammonium hydroxide is used as catalyst, based on the amount of diisocyanate used.

2. Process according to Claim 1, **characterized in that** the catalyst solution is metered in such that the reaction temperature is > 90°C and ≤ 135°C.

3. Process according to Claim 1, **characterized in that** the catalyst solution is metered in such that the reaction temperature is ≥ 95°C and ≤ 120°C.

4. Process according to any of Claims 1 to 3, **characterized in that** this process is carried out continuously in one or more stirred tanks.

5. Process according to any of Claims 1 to 4, **characterized in that** an NCO content is achieved corresponding to a degree of trimerisation of 8 to 30%.

6. Continuous process for preparing polyisocyanates containing isocyanurate groups by trimerising cycloaliphatic diisocyanates using a catalyst solution comprising at least one quaternary ammonium hydroxide in a stirred tank cascade with n stirred tanks, **characterized in that**, in at least n/2 or (n/2 - 0.5) tanks, the metered addition is such that the reaction temperature in at least n/2 or (n/2 + 0.5) tanks is > 90°C and ≤ 140°C, where n/2 applies to an even number of tanks and (n/2 - 0.5) or (n/2 + 0.5) applies to an odd number of tanks and n is a whole number from 2 to 6.

7. Process according to Claim 6, **characterized in that** an NCO content is achieved in the nth tank corresponding to a degree of trimerisation of 8 to 30%.

8. Process according to any of Claims 1 to 7, **characterized in that** the catalysts are used as solutions at concentrations of 0.1 - 10% by weight, based on the catalyst solution.

9. Process according to any of Claims 1 to 8, **characterized in that** at least one quaternary tetraalkylammonium hydroxide and/or a mixed quaternary tetraalkyl/aralkylammonium hydroxide and/or a quaternary tetraaralkylammonium hydroxide, is used as catalyst.

10. Process according to Claim 9, **characterized in that** at least one quaternary trialkylaralkylammonium hydroxide is used as catalyst.

11. Process according to Claim 9, **characterized in that** at least one quaternary tetraalkylammonium hydroxide and/or one quaternary mixed tetraalkyl/aralkylammonium hydroxide is used as catalyst, the alkyl radicals of which do not comprise any hydroxyl groups.

12. Process according to Claim 9, **characterized in that** a quaternary benzyltrialkylammonium hydroxide is used exclusively as catalyst.

13. Process according to Claim 9, **characterized in that** benzyltrimethylammonium hydroxide is used exclusively as catalyst.

14. Process according to any of Claims 1 to 13, **characterized in that** IPDI is used as cycloaliphatic diisocyanate.

15. Process according to any of Claims 1 to 14, **characterized in that** the reaction solution is stopped chemically or thermally, preferably thermally.

16. Process according to Claim 15, **characterized in that** the reaction solution is stopped chemically by addition of an acidic compound, an acid and/or an alkylating agent.

17. Process according to any of Claims 1 to 16, **characterized in that**, in a downstream step, monomeric cycloaliphatic diisocyanate still present is removed from the polyisocyanate by distillation.

## Revendications

1. Procédé de fabrication de polyisocyanates contenant des groupes isocyanurate par trimérisation de diisocyanates cycloaliphatiques en utilisant une solution de catalyseur contenant au moins un hydroxyde d'ammonium quaternaire, **caractérisé en ce que** la solution de catalyseur est dosée de telle sorte que la température de réaction soit > 90 °C et ≤ 140 °C, le 1,3- ou 1,4-diisocyanatocyclohexane, le 1,4-diisocyanato-3,3,5-triméthylcyclohexane, le 1,3-diisocyanato-2(4)-méthylcyclohexane, le 1-isocyanato-3,3,5-triméthyl-5-isocyanatométhylcyclohexane (diisocyanate d'isophorone, IPDI), le 1-isocyanato-1-méthyl-4(3)isocyanatométhylcyclohexane, le 1,8-diisocyanato-p-menthane, le 4,4'-diisocyanato-1,1 '-bi(cyclohexyle), le 4,4'-diisocyanato-3,3'-diméthyl-1,1'-bi(cyclohexyle), le 4,4'-diisocyanato-2,2',5,5'-tétraméthyl-1,1'-bi(cyclohexyle), le 4,4'-diisocyanatodicyclo-hexylméthane, le 2,4'-diisocyanatodicyclohexylméthane, le 4,4'-diisocyanato-3,3'-diméthyldicyclohexylméthane, le 4,4'-diisocyanato-3,3',5,5'-tétraméthyl-dicyclohexylméthane, le 1,3-diisocyanato-adamantane, le 1,3-diméthyl-5,7-diisocyanatoadamantane, ainsi que des mélanges quelconques de ces diisocyanates étant utilisés en tant que diisocyanates cycloaliphatiques, et 0,005 à 1 % en poids, par rapport à la quantité de diisocyanate utilisé, d'hydroxyde d'ammonium quaternaire étant utilisé en tant que catalyseur.

2. Procédé selon la revendication 1, **caractérisé en ce que** la solution de catalyseur est dosée de telle sorte que la température de réaction soit > 90 °C et ≤ 135 °C.

3. Procédé selon la revendication 1, **caractérisé en ce que** la solution de catalyseur est dosée de telle sorte que la température de réaction soit ≥ 95 °C et ≤ 120 °C.

4. Procédé selon l'une quelconque des revendications 1 à 3, **caractérisé en ce que** celui-ci est réalisé en continu dans une ou plusieurs cuves agitées.

5. Procédé selon l'une quelconque des revendications 1 à 4, **caractérisé en ce qu'**une teneur en NCO qui correspond à un degré de trimérisation de 8 à 30 % est atteinte.

6. Procédé continu de fabrication de polyisocyanates contenant des groupes isocyanurate par trimérisation de diisocyanates cycloaliphatiques en utilisant une solution de catalyseur contenant au moins un hydroxyde d'ammonium quaternaire dans une cascade de cuves agitées contenant n cuves agitées, **caractérisé en ce que** le dosage est réalisé de telle sorte dans au moins n/2 ou (n/2 - 0,5) cuves que la température de réaction dans au moins n/2 ou (n/2 + 0,5) cuves soit > 90 °C et ≤ 140 °C, n/2 s'appliquant pour un nombre pair de cuves et (n/2 - 0,5) et (n/2 + 0,5) s'appliquant pour un nombre impair de cuves, et n signifiant un nombre entier de 2 à 6.

7. Procédé selon la revendication 6, **caractérisé en ce qu'**une teneur en NCO qui correspond à un degré de trimérisation de 8 à 30 % est atteinte dans la n^{e} cuve.

8. Procédé selon l'une quelconque des revendications 1 à 7, **caractérisé en ce que** les catalyseurs sont utilisés sous la forme de solutions d'une concentration de 0,1 à 10 % en poids, par rapport à la solution de catalyseur.

9. Procédé selon l'une quelconque des revendications 1 à 8, **caractérisé en ce qu'**au moins un hydroxyde de tétraalkylammonium quaternaire et/ou un hydroxyde de tétraalkyl/aralkylammonium quaternaire mixte et/ou un hydroxyde de tétraaralkylammonium quaternaire sont utilisés en tant que catalyseur.

10. Procédé selon la revendication 9, **caractérisé en ce qu'**au moins un hydroxyde de trialkylaralkylammonium quaternaire est utilisé en tant que catalyseur.

11. Procédé selon la revendication 9, **caractérisé en ce qu'**au moins un hydroxyde de tétraalkylammonium quaternaire et/ou un hydroxyde de tétraalkyl/aralkylammonium mixte quaternaire sont utilisés en tant que catalyseur, dont les radicaux alkyle ne contiennent pas de groupes hydroxyle.

12. Procédé selon la revendication 9, **caractérisé en ce qu'**exclusivement un hydroxyde de benzyltrialkylammonium quaternaire est utilisé en tant que catalyseur.

13. Procédé selon la revendication 9, **caractérisé en ce qu'**exclusivement un hydroxyde de benzyltriméthylammonium est utilisé en tant que catalyseur.

14. Procédé selon l'une quelconque des revendications 1 à 13, **caractérisé en ce que** l'IPDI est utilisé en tant que diisocyanate cycloaliphatique.

15. Procédé selon l'une quelconque des revendications 1 à 14, **caractérisé en ce que** la solution de réaction est arrêtée chimiquement ou thermiquement, de préférence thermiquement.

16. Procédé selon la revendication 15, **caractérisé en ce que** la solution de réaction est arrêtée chimiquement par ajout d'un composé acide, d'un acide et/ou d'un agent d'alkylation.

17. Procédé selon l'une quelconque des revendications 1 à 16, **caractérisé en ce que** le diisocyanate cycloaliphatique monomère encore présent est séparé par distillation du polyisocyanate lors d'une étape en aval.
